(19) Europäisches Patentamt
European Patent Office
Office européen des brevets

(11) Veröffentlichungsnummer : **0 164 763**
**B1**

(12) # EUROPÄISCHE PATENTSCHRIFT

(45) Veröffentlichungstag der Patentschrift : **16.06.87**

(51) Int. Cl.⁴ : **C 07 C121/00**, C 07 C 69/753, C 07 C 49/553, C 11 B 9/00, A 23 L 1/226, A 61 K 7/46

(21) Anmeldenummer : **85108929.2**

(22) Anmeldetag : **18.07.80**

(60) Veröffentlichungsnummer der früheren Anmeldung nach Art. 76 EPÜ : **0024306**

(54) **Neue substituierte Tetraline und Indane (I), Verwendung von (I) als Riech- und/oder Geschmackstoffe sowie Riech- und/oder Geschmackstoffkompositionen mit einem Gehalt an (I).**

(30) Priorität : **10.08.79 CH 7355/79**
***30.06.80 CH 5003/80***

(43) Veröffentlichungstag der Anmeldung : **18.12.85 Patentblatt 85/51**

(45) Bekanntmachung des Hinweises auf die Patenterteilung : **16.06.87 Patentblatt 87/25**

(84) Benannte Vertragsstaaten : **CH DE FR GB LI NL**

(56) Entgegenhaltungen :
**FR-A- 2 239 997**
**FR-A- 2 348 916**
**US-A- 3 910 853**

(73) Patentinhaber : **L. Givaudan & Cie Société Anonyme**
***Patentdienst Postfach***
**CH-4002 Basel (CH)**

(72) Erfinder : **Gonzenbach, Hans Ulrich, Dr.**
**61bis rue de Lyon**
**CH-1203 Genf. (CH)**
Erfinder : **Ochsner, Paul Albert, Dr.**
**30 avenue des Tilleuls**
**CH-1203 Genf. (CH)**

(74) Vertreter : **Urech, Peter, Dr. et al**
**Grenzacherstrasse 124 Postfach 3255**
**CH-4002 Basel (CH)**

**EP 0 164 763 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Riech- und/oder Geschmackstoffkompositionen. Diese sind gekennzeichnet durch einen Gehalt an einer Verbindung der Formel

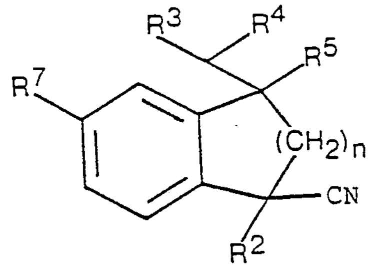

(I)

worin die Reste $R^2$ bis $R^8$ für Wasserstoff oder Methyl stehen und n = 1 oder 2 ist.

Die Formel soll demgemäss die Tetralinderivate Ia und die Indanderivate Ib repräsentieren :

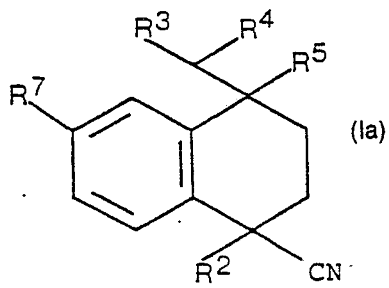

(Ia)

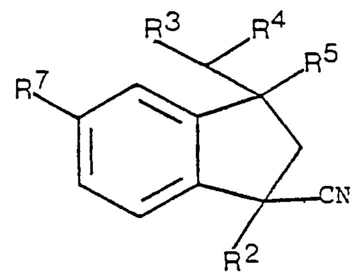

(Ib)

worin $R^2$ bis $R^8$ obige Bedeutung besitzen.

Die Verbindungen der Formel Ia sind bevorzugt.

Diese Verbindungen der Formel Ia sind neu. Die Verbindungen der Formel Ib sind, mit Ausnahme des 1,1-Dimethyl-3-cyanoindans, ebenfalls neu. Die Erfindung betrifft demgemäss auch die neuen Verbindungen der Formel I.

Die Verbindungen I weisen besondere organoleptische Eigenschaften auf, auf Grund derer sie sich vorzüglich als Riech- und/oder Geschmackstoffe eignen.

Die Erfindung betrifft demgemäss auch die Verwendung der Verbindungen I als Riech- und/oder Geschmackstoffe.

Die Verbindungen der Formel I eignen sich aufgrund ihrer natürlichen Geruchsnoten und ihrer Haftdauer (Langzeiteffekt, insbesondere bezüglich Frische) insbesondere zur Modifizierung von bekannten, z. B.

α) blumigen Kompositionen, in denen z. B. die Citrusnoten verstärkt zum Ausdruck kommen sollen (z. B. für Cologne-Typen u. ä., Extraits),

β) des weiteren aber auch von fruchtigen, z. B. Typ Himbeere (Extrait-Typen, Komposition der femininen Richtung), von

γ) Tabak- und Holzkompositionen, (Extrait-Typen der masculinen Richtung) und schliesslich von

δ) Kompositionen mit grünen Noten, wo insbesondere eine erwünschte Abrundung und harmonisierende Effekte erzielt werden.

Besonders interessante Verbindungen sind :

1,1-Dimethyl-4-cyano-tetralin : angenehm fruchtiger, tabakartiger Geruch, nach Stroh mit einem Anklang an Patchouli und Ambre. In Kompositionen werden unerwartete Effekte gefunden. 1,1-Dimethyl-4-cyano-tetralin verleiht diesen Volumen und rundet sie auf harmonische Weise ab. Es wird eine Natürlichkeit « erzielt », die bei rein synthetischen Kompositionen äusserst willkommen ist. Gleichzeitig werden die Kopfnoten fixiert, so dass die Kompositionen länger ausgeglichen wirken.

1,1,7-Trimethyl-4-cyano-tetralin : wie obige Verbindung, aber nobler wirkend und mit leichtem Moschuscharakter.

Die Verbindungen I verbinden sich mit zahlreichen bekannten Riech- und/oder Geschmackstoffingredienten natürlichen oder synthetischen Ursprungs, wobei die Palette der natürlichen Rohstoffe sowohl leicht- als auch mittel- und schwerflüchtige Komponenten, und diejenige der Synthetika Vertreter aus praktisch allen Stoffklassen umfassen kann, wie dies aus der folgenden Zusammenstellung ersichtlich ist :

Naturprodukte, wie Baummoos-Absolue, Basilikumöl, Bergamottöl, acetyliertes Cedernholzöl (z. B. Vertofix ®IFF bzw. Cedartone ®Givaudan), Eichenmoos, Galbanumöl, Geraniumöl, Jasmin Absolue und

seine Substitute, Lavendelöl, Lavandinöl, Mastix Absolue, Neroliöl, Patchouliöl, Petitgrainöl Paraguay, Sandelholzöl, Vetiveröl, Ylang-Ylang-Oel, Zitronenöl, Wermutöl,

Alkohole, wie Linalool, Citronellol, Geraniol, natürliches Rhodinol, α-Terpineol, Phenyläthylalkohol, Phenylpropylalkohol, Zimtalkohol, 3-Methyl-5-(2',2',3'-trimethyl-cyclopent-3'-en-1'-yl)-pentan-2-ol (Sandalore ®Givaudan),

Aldehyde, wie 3,5-Dimethyl-cyclohex-3-en-carboxaldehyd, Decanal, Methylnonylacetaldehyd, Hydroxycitronellal, α-Hexylzimtaldehyd, Cyclamenaldehyd, p-tert. Butyl-α-methyl-dihydro-zimt-aldehyd (z. B. Lilial ®Givaudan), Citral,

Ketone, wie α-Jonon, Acetylcedren, p-Methylacetophenon, Methyljonone,

Ester, wie Cedrylacetat, cis-3-Hexenylacetat, cis-3-Hexenylbenzoat, Aethylacetoacetat, Linalylacetat, Geranylacetat, Terphenylacetat, Phenyläthylacetat, Styrallylacetat, p-tert. Butylcyclohexylacetat, 4[4-Methyl-3-pentenyl-]-cyclohex-3-en-1-yl-carbinylacetat (z. B. Myraldylacetat ®Givaudan), Cinnamylformiat, Benzylacetat, Benzylsalicytat, Amylsalicylat, Methyldihydrojasmonat, 3-Aethyl-1,1-dimethylcyclohex-3-en-2-carbonsäureäthylester (Givescone ®Givaudan),

Lactone, wie γ-Undecalacton, Cumarin,

verschiedene weitere, in der Parfümerie oft benützten Komponenten wie Moschus-Verbindungen (Ambrette-, Ketonmoschus, 12-Oxa-hexadecanolid (z. B. Musk 174 ®Naarden), 1,1-Dimethyl-4-acetyl-6-tert.butylindan, Indol), p-Methan-8-thiol-3-on, Eugenol, Acetaldehyd-propylphenyl-äthylacetal, Methyl-1-methyl-cyclododecyläther (z. B. Madrox ®Givaudan).

Die Verbindungen der Formel I lassen sich in weiten Grenzen einsetzen, die beispielsweise von 0,1 (Detergentien) — 50 % (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0,5 und 25 %. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Tabak, etc.).

Die Verbindungen I können demgemäss bei der Herstellung von Kompositionen und — wie obige Zusammenstellung zeigt — unter Verwendung einer breiten Palette bekannter Riechstoffe, verwendet werden. Bei der Herstellung solcher Kompositionen können die oben aufgeführten bekannten Riechstoffe nach (dem Parfümer bekannter) Art und Weise verwendet werden, wie z. B. aus W. A. Poucher, Perfumes, Cosmetics and Soaps 2, 7. Auflage, Chapman und Hall, London, 1974 hervorgehend.

Als Geschmackstoffe können die Verbindungen I beispielsweise zur Erzeugung bzw. Verbesserung, Verstärkung, Steigerung oder Modifizierung von Frucht- oder Beerenaromen, insbesondere Himbeeraromen, in Nahrungsmitteln (Joghurt, Süsswaren, etc.) ; in Genussmitteln (Tee, Tabak, etc.) und Getränken (Limonaden etc.) verwendet werden.

Die ausgeprägten geschmacklichen Qualitäten der Verbindungen I ermöglichen die Verwendung in geringen Konzentrationen. Eine geeignete Dosierung umfasst beispielsweise den Bereich von 0,01 ppm-100 ppm, vorzugsweise von 0,1 ppm-20 ppm im Fertigprodukt, d. h. dem aromatisierten Nahrungsmittel, Genussmittel oder Getränk.

Bei der Aromatisierung von beispielsweise Tabak kann die Dosierung jedoch auch höher liegen und einen grösseren Bereich umfassen, beispielsweise den Bereich von 1 bis 1 000 ppm, vorzugsweise 50-500 ppm.

Die Verbindungen können auf übliche Weise mit den für Geschmackstoffkompositionen verwendeten Bestandteilen vermischt bzw. solchen Aromen zugesetzt werden. Unter den erfindungsgemäss verwendeten Aromen werden Geschmackstoffkompositionen verstanden, die sich auf an sich bekannte Art verdünnen bzw. in essbaren Materialien verteilen lassen. Sie enthalten beispielsweise etwa 0,1-10, insbesondere 0,5-3 Gew.%. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammenstellung bereits enthalten oder können leicht der Literatur entnommen werden, wie z. B. J. Merory, Food Flavorings, Composition, Manufacture and Use, Second Edition, The Avi Publishing Company, Inc., Westport, Conn. 1968, oder G. Fenaroli, Fenaroli's Handbook of Flavor Ingredients, Second Edition, Volume 2, CRC-Press, Inc. Cleveland, Ohio, 1975.

Für die Herstellung solcher üblicher Gebrauchsformen kommen beispielsweise folgende Trägermaterialien, Verdickungsmittel, Geschmackstoffverbesserer, Gewürze und Hilfsingredientien, etc. in Frage :

Gummi arabicum, Tragant, Salze oder Brauereihefe, Alginate, Carrageen oder ähnliche Absorbentien ; Indole, Maltol, Dienale, Gewürzoleoresine, Raucharomen ; Gewürznelken, Diacetyl, Natriumcitrat ; Mononatriumglutamat, Dinatriuminosin-5'-monophosphat (IMP), Dinatriumguanosin-5-phosphat (GMP) ; oder spezielle Aromastoffe, Wasser, Aethanol, Propylenglykol, Glycerin.

Die Verbindungen der Formel I sind beispielsweise gemäss Reaktionsschema I zugänglich. Bei den dort aufgeführten Methoden handelt es sich um an sich bekannte Verfahren ; es sind dies insbesondere (siehe auch Tabelle I) :

ⓐ Alkenylierungen
ⓑ Alkylierungen
ⓒ Cyclisierungen

Die Tabelle I und Schema I informieren über zweckmässige Methoden und Mittel sowie zweckmässige und bevorzugte Reaktionsparameter der einzelnen Verfahrensvarianten.

Schema I

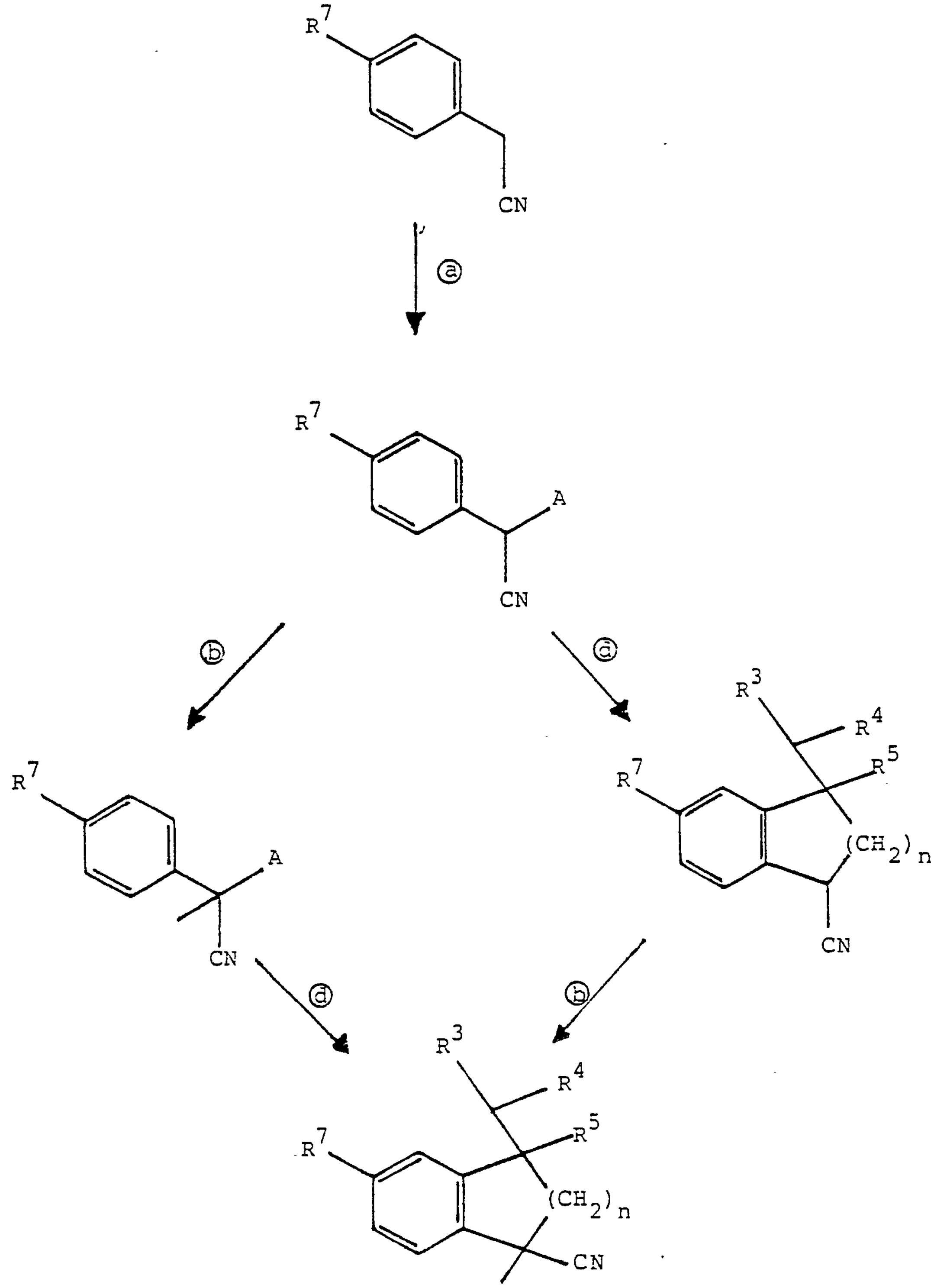

Tabelle I

| Reaktion | Edukt | Produkt | ReagenS | Reaktionsbedingungen Lsm/Kat./etc. | Bemerkungen / Literatur |
|---|---|---|---|---|---|
| (a) Alkenylierung | 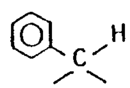 | 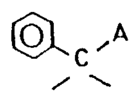 | XA, XA' | a) unter Phasentransferbed. | Jozef Dockx, Synthesis 1973, 441 |
| (b) Methylierung | 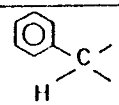 | 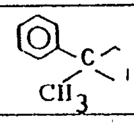 | $XCH_3$ | b) klassisch z.B. +OH/+OK EtOH/EtONa $Et_2O$/NaH etc. | ORGANIKUM, VEB, Deutscher Verlag der Wissenschaften, Berlin, 1967 |
| (d) Cyclisierung | 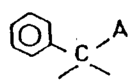 | 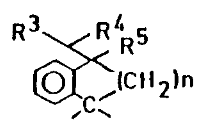 | PPS $H_3PO_4$ 85% | "mild" 50-100°C | Cyclisierung gelingt unter Erhaltung der Nitrilfunktion, im Gegensatz zu E. GROCHOWSKI + T. BOLESLAWSKA Bull. Acad. Polon. Sci., Ser. Sci. Chim. 20, 297 (1972) |

Legende zu Tabelle I und Schema I

A = 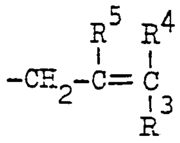 , 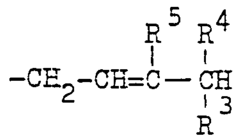 , 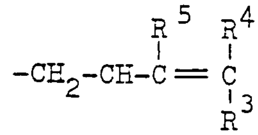

A' = 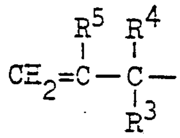 , 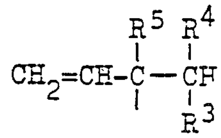

X = Halogen (-Cl, -Br, -J)
PPS = Polyphosphorsäure
es bedeuten

. . . . } einzeln oder Gemisch
. . . .

Lsm = Lösungsmittel

## Beispiel 1

Herstellung der Ausgangsmaterialien :

A) 5-Methyl-2-phenyl-4-hexennitril

In einem 2-Liter-Kolben, versehen mit Thermometer, Rührer und Rückflusskühler werden 384 g 50 %ige Natronlauge (4,8 Mol) und 12 g Hexadecyl-trimethylammoniumbromid vorgelegt und unter Rühren innert einer Viertelstunde ein Gemisch von 469 g Benzylcyanid (4 Mol) und 502 g Prenylchlorid [1)] (4,8 Mol) zugetropft ; während der Zugabe erwärmt sich das Gemisch, es wird anschlissend noch während 3 Stunden bei 60 °C weitergerührt. Nach Abkühlung wird auf 500 g Eis gegossen, mit Aether ausgeschüttelt (4 x) und mit Wasser neutral gewaschen. Die Phasentrennung kann durch die Zugabe von wenig Alkohol erleichtert werden. Nach Trocknung über Natriumsulfat wird der Aether abgedampft und fraktioniert destilliert. Man erhält 444 g 5-Methyl-2-phenyl-4-hexennitril, Sdp. 108-109 °C/3 mmHg, $n_D^{20}$ : 1,5171-72, Ausbeute 60 %.

B) 4-Methyl-2-phenyl-4-pentennitril

Wird in obigem Verfahren anstelle von Prenylchlorid β-Methallylchlorid verwendet, erhält man 4-Methyl-2-phenyl-4-pentennitril, Sdp. 72 °C/0,13 mmHg, $n_D^{20}$ : 1,5203.

C) 5-Methyl-2-(p-tolyl)-4-hexennitril

Aus p-Methyl-benzylcyanid und Prenylchlorid erhält man in analoger Weise 5-Methyl-2-(p-tolyl)-4-hexennitril, Sdp. 89 °C/0,03 mmHg, $n_D^{20}$ : 1,5176.

## Beispiel 2

Verbindungen der Formel I :

1) 1,1-Dimethyl-4-cyano-tetralin

46,3 g 85 %ige Phosphorsäure, 46,3 g Polyphosphorsäure, 250 ml Toluol und 92,6 g 5-Methyl-2-phenyl-4-hexennitril werden zusammen gegeben und unter Rühren während 3 Stunden auf 100 °C erwärmt ; das Gemisch wird hierauf auf Eis gegossen. Es wird nun mit Aether extrahiert, mit Wasser, mit 10 %iger Natriumcarbonatlösung und erneut mit Wasser bis zum Neutralpunkt gewaschen, getrocknet, eingedampft und im Vakuum destilliert. Es werden 85,3 g 1,1-Dimethyl-4-cyano-tetralin erhalten, Sdp. 114 °C/2 mm, $n_D^{20}$ : 1,5351. Ausbeute 92 %. Geruch : angenehm fruchtig, tabakartig, nach Stroh mit Anklang an Patchouli und Ambra.

[1)] Gemisch von 1-Chlor-3-methyl-2-buten und 3-Chlor-3-methyl-1-buten

1bis) 1,1,7-Trimethyl-4-cyano-tetralin

Aus 5-Methyl-2-(p-tolyl)-4-hexen-nitril erhält man analog 1,1,7-Trimethyl-4-cyano-tetralin, Sdp. 89 °C (0,02 mmHg, $n_D^{20}$ : 1,5340. Geruch : ähnlich 1,1-Dimethyl-4-propionyltetralin : nämlich Rosennote, tabakartig (blond), Trockenfrüchte, Beeren, Damascone, Honig, aber mehr nach Rosen und Tee.

2) 1,1-Dimethyl-3-cyano-indan

4-Methyl-2-phenyl-4-pentennitril liefert bei analoger Behandlung mit Polyphosphorsäure 1,1-Dimethyl-3-cyano-indan, Sdp. 82 °C/0,04 mmHg, $n_D^{20}$ : 1,5248. Geruch : erdig.

3) 1,1,4-Trimethyl-4-cyano-tetralin

81,5 g 1,1-Dimethyl-4-cyano-tetralin werden zu 49 g Kalium-tert.butylat in 400 ml tert.-Butanol zugetropft und es wird 1 Stunde bei Raumtemperatur gerührt. Bei 5 °C werden darauf 72 g Methyljodid zugetropft und die Mischung 15 Stunden ohne Kühlung gerührt. Die Hauptmenge des tert.-Butanols wird abdestilliert, der Rest in Aether aufgenommen und mit Wasser neutral gewaschen, über Natriumsulfat getrocknet und eingedampft. Man destilliert im Vakuum und erhält 61 g 1,1,4-Trimethyl-4-cyano-tetralin, Sdp. 85 °C/0,25 mmHg, Smp. 46 °C, Ausbeute 70 %. Geruch : ähnlich 1,1-Dimethyl-4-propionyl-tetralin, aber intensiver, holzig.

Beispiel 4

In den folgenden Formulierungsbeispielen steht V für 1,1-Dimethyl-4-cyano-tetralin.

1. Rosenbase

| A : | Gewichtsteile |
|---|---|
| Phenyläthanol | 200 |
| Citronellol extra | 150 |
| Geraniol pur | 150 |
| Rhodinol pur | 80 |
| Ambroxane $^R$(8α, 12-Oxido-13,14,15,16-tetranorlabdan) | 30 |
| Fixolide $^R$(6-Acetyl-1,1,2,4,4,7-hexamethyl-1,2,3,4-tetrahydronaphthalin) | 100 |
| Lemarome N$^R$ (Gemisch von 1/3 Neral, 2/3 Geranial) | 30 |
| Phenylessigsäure | 5 |
| Dipropylenglykol | 155 |
| | 900 |

B : A mit Zusatz von 100 Gewichtsteilen V.

Olfaktorische Beurteilung :

B wirkt im Vergleich zu A bedeutend reicher, hat mehr Volumen, ist weniger trocken und dafür mehr honigartig. B hat, obwohl nur aus synthetischen Riechstoffen bestehend, einen ausgeprägt natürlichen Charakter und erinnert an Teerosen.

2. Citrusbase

| A : | Gewichtsteile |
|---|---|
| Linalylacetat synth. | 200 |
| Lemarone N$^R$ | 150 |
| Laurin (Hydroxycitronellal extra) | 100 |
| Linalool synth. | 200 |
| $C_{11}$-Aldehyd (10 % in Carbitol) | 10 |
| Xylolmoschus | 20 |
| Fixolide $^R$ | 50 |
| Dipropylenglykol | 170 |
| | 900 |

B : dito mit Zusatz von 100 Gewichtsteilen V.

Olfaktorische Beurteilung :

Der Zusatz von V verleiht der Komposition A einen natürlichen Charakter, wie er bei rein synthetischen Kompositionen sonst nur schwer zu erzielen ist. B wirkt zudem reicher und wärmer und ist harmonisch völlig abgerundet.

3. Citrusbase

| A : | Gewichtsteile |
|---|---|
| Linalylacetat synth. | 200 |
| Lemarone N[R] | 150 |
| Laurin (Hydroxycitronellal extra) | 100 |
| Linalool synth. | 200 |
| $C_{11}$-Aldehyd (10 % in Carbitol) | 10 |
| Xylolmoschus | 20 |
| Fixolide[R] | 50 |
| Dipropylenglykol | 100 |
| Bergamotteöl | 70 |
| | 900 |

B : dito mit Zusatz von 100 Gewichtsteilen V.

Olfaktorische Beurteilung :

A entspricht bzgl. natürlichem Charakter im wesentlichen der Komposition 2B, denn durch das Bergamotteöl wurde eine natürliche Note erzielt. Trotzdem wird 3B gegenüber 3A eindeutig bevorzugt, weil die Kopfnoten fixiert werden und B so eine natürliche Harmonie wesentlich länger beibehält.

4. Tuberosen-Base

| A : | Gewichtsteile |
|---|---|
| α-Hexyl-zimtaldehyd | 140 |
| Laurin (Hydroxycitronellal extra) | 120 |
| Linalool synth. | 100 |
| Terpineol | 100 |
| Prunolide (γ-Nonalacton) (10 % in Dipropylenglykol) | 20 |
| Peche pur (γ-Undecalacton) (10 % in Dipropylenglykol) | 20 |
| Tolubalsam | 140 |
| Jasmin absolut | 40 |
| Isoeugenol | 40 |
| Phenylacetaldehyd (10 % in Dipropylenglykol) | 40 |
| Fixolide[R] | 100 |
| Dipropylenglykol | 40 |
| | 900 |

B : dito mit Zusatz von 100 Gewichtsteilen V.

Olfaktorische Beurteilung :

B wirkt fruchtiger, blumiger, reicher als A und im Vergleich zu diesem natürlich abgerundet. Nach 6 Stunden hat B seine natürliche Frische bewahrt, während A nur noch platt wirkt.

**Patentansprüche**

1. Riech- und/oder Geschmackstoffkomposition, gekennzeichnet durch einen Gehalt an einer Verbindung der Formel

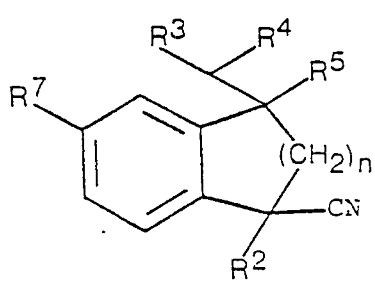

(I)

worin die Reste $R^2$ bis $R^8$ für Wasserstoff oder Methyl stehen und n = 1 oder 2 ist.

2. Riech- und/oder Geschmackstoffkomposition, gekennzeichnet durch einen Gehalt an einer Verbindung der allgemeinen Formel

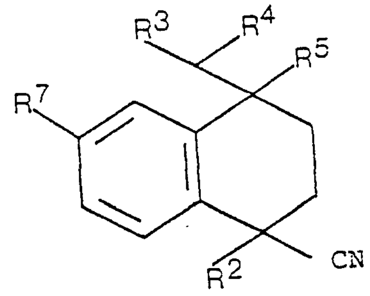

(Ia)

worin die Reste $R^2$ bis $R^8$ für Wasserstoff oder Methyl stehen.

3. Riech- und/oder Geschmackstoffkomposition, gekennzeichnet durch einen Gehalt an einer Verbindung der allgemeinen Formel

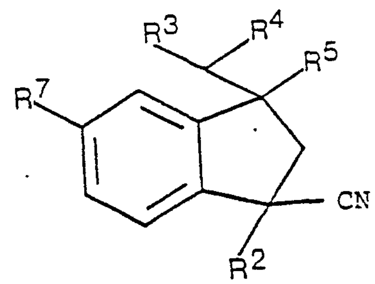

(Ib)

worin die Reste $R^2$ bis $R^8$ für Wasserstoff oder Methyl stehen.

4. Riech- und/oder Geschmackstoffkomposition, gekennzeichnet durch einen Gehalt an 1,1-Dimethyl-4-cyanotetralin.

5. Riech- und/oder Geschmackstoffkomposition, gekennzeichnet durch einen Gehalt an 1,1,7-Trimethyl-4-cyanotetralin.

6. Verwendung von Verbindungen der Formel

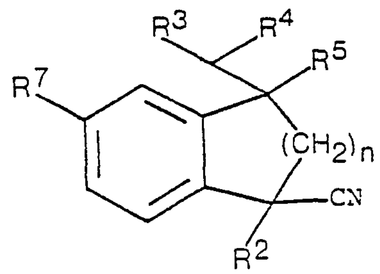

(I)

worin die Reste $R^2$ bis $R^8$ für Wasserstoff oder Methyl stehen und n = 1 oder 2 ist, als Riech- und/oder Geschmackstoffe.

7. Verbindungen der Formel

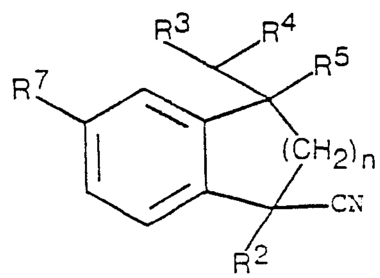

(I)

worin die Reste $R^2$ bis $R^8$ für Wasserstoff oder Methyl stehen und n = 1 oder 2 ist, mit der Ausnahme des 1,1-Dimethyl-3-cyanoindans.

8. Verbindungen der Formel

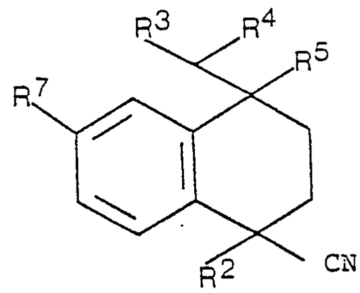

(Ia)

worin die Reste $R^2$ bis $R^8$ für Wasserstoff oder Methyl stehen.

9. 1,1-Dimethyl-4-cyano-tetralin.

10. 1,1,7-Trimethyl-4-cyano-tetralin.

11. Verbindungen der Formel

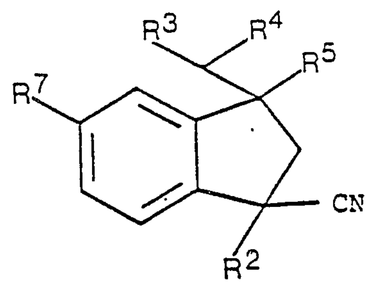

(Ib)

worin die Reste $R^2$ bis $R^8$ für Wasserstoff oder Methyl stehen, mit der Ausnahme des 1,1-Dimethyl-3-cyanoindans.

12. 1,1,4-Trimethyl-4-cyano-tetralin.

**Claims**

1. An odorant and/or flavouring composition, characterized by a content of a compound of the formula

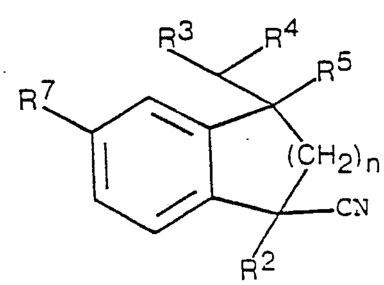

(I)

wherein the residues $R^2$ to $R^8$ stand for hydrogen or methyl and n = 1 or 2.

2. An odorant and/or flavouring composition, characterized by a content of a compound of the general formula

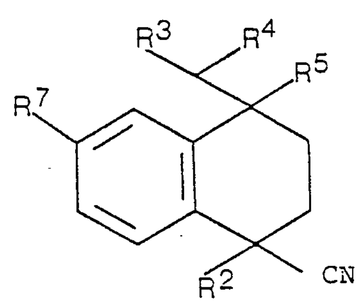

(Ia)

wherein the residues $R^2$ to $R^8$ stand for hydrogen or methyl.

3. An odorant and/or flavouring composition, characterized by a content of a compound of the general formula

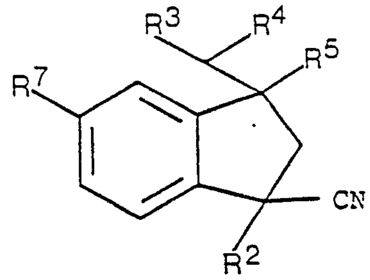

(Ib)

wherein the residues $R^2$ to $R^8$ stand for hydrogen or methyl.

4. An odorant and/or flavouring composition, characterized by a content of 1,1-dimethyl-4-cyanotetralin.

5. An odorant and/or flavouring composition, characterized by a content of 1,1,7-trimethyl-4-cyanotetralin.

6. The use of compounds of the formula

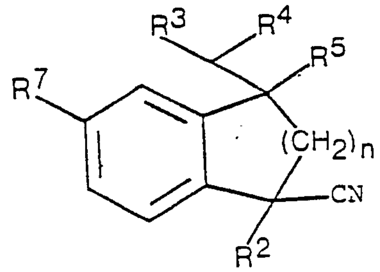

(I)

wherein the residues $R^2$ to $R^8$ stand for hydrogen or methyl and n = 1 or 2, as odorant and/or flavouring substances.

7. Compounds of the formula

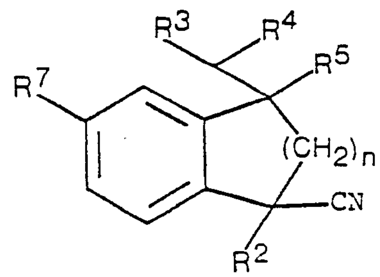

(I)

wherein the residues $R^2$ to $R^8$ stand for hydrogen or methyl and n = 1 or 2, with the exception of 1,1-dimethyl-3-cyanoindane.

8. Compounds of the formula

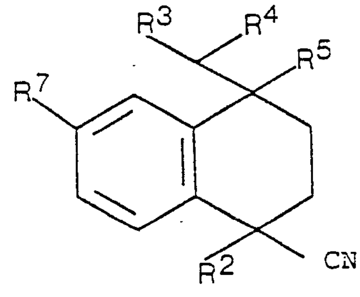

(Ia)

wherein the residues $R^2$ to $R^8$ stand for hydrogen or methyl.

9. 1,1-Dimethyl-4-cyano-tetralin.

10. 1,1,7-Trimethyl-4-cyano-tetralin.

11. Compounds of the formula

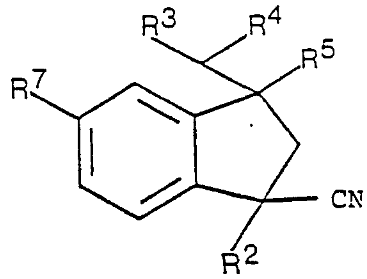

(Ib)

wherein the residues $R^2$ to $R^8$ stand for hydrogen or methyl, with the exception of 1,1-dimethyl-3-cyanoindane.

12. 1,1,4-Trimethyl-4-cyano-tetralin.

## Revendications

1. Composition odorante et/ou aromatisante caractérisée en ce qu'elle contient un composé de formule

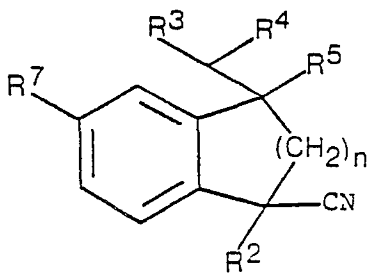

(I)

où les radicaux $R^2$ à $R^8$ représentent un hydrogène ou un méthyle et n = 1 ou 2.

2. Composition odorante et/ou aromatisante caractérisée en ce qu'elle contient un composé de formule générale

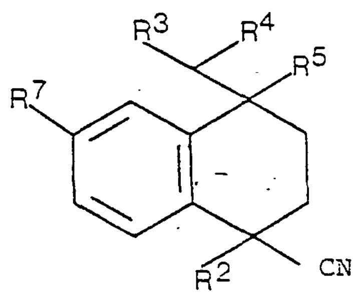

(Ia)

où les radicaux $R^2$ à $R^8$ représentent un hydrogène ou un méthyle.

3. Composition odorante et/ou aromatisante caractérisée en ce qu'elle contient un composé de formule générale

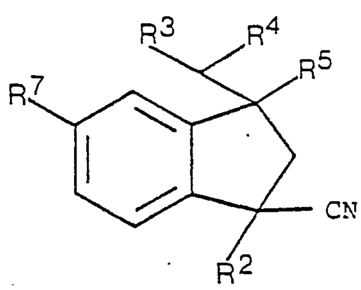

(Ib)

où les radicaux $R^2$ à $R^8$ représentent un hydrogène ou un méthyle.

4. Composition odorante et/ou aromatisante caractérisée en ce qu'elle contient la 1,1-diméthyl-4-cyanotétraline.

5. Composition odorante et/ou aromatisante caractérisée en ce qu'elle contient la 1,1,7-triméthyl-4-cyanotétraline.

6. Application de composés de formule

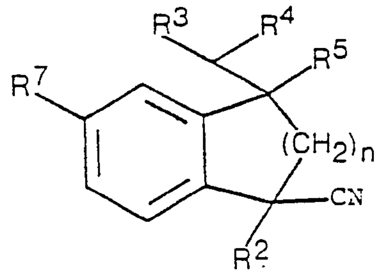

(I)

où les radicaux $R^2$ à $R^8$ représentent un hydrogène ou un méthyle et n = 1 ou 2, comme agents odorants et/ou aromatisants.

7. Composés de formule

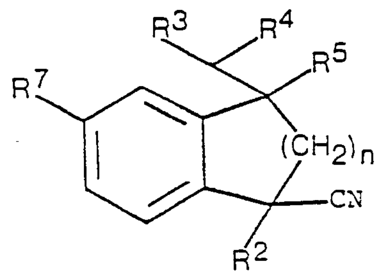

(I)

où les radicaux $R^2$ à $R^8$ représentent un hydrogène ou un méthyle et n = 1 ou 2, à l'exception du 1,1-diméthyl-3-cyanoindane.

8. Composés de formule

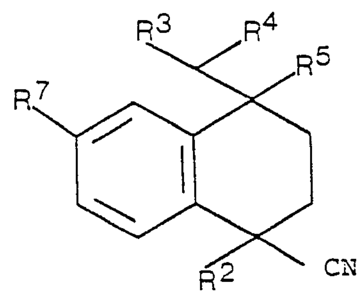

(Ia)

où les radicaux $R^2$ à $R^8$ représentent un hydrogène ou un méthyle.

9. 1,1-diméthyl-4-cyano-tétraline.

10. 1,1,7-triméthyl-4-cyano-tétraline.

11. Composés de formule

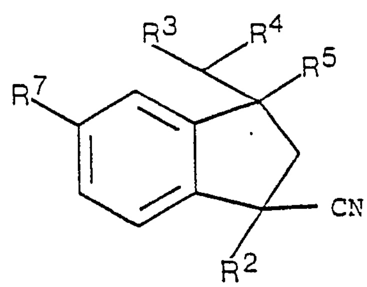

(Ib)

où les radicaux $R^2$ à $R^8$ représentent un hydrogène ou un méthyle, à l'exception du 1,1-diméthyl-3-cyanoindane.

12. 1,1,4-triméthyl-4-cyano-tétraline.